# EUROPEAN PATENT APPLICATION

(11) **EP 2 078 746 A1**
(43) Date of publication of application: **15.07.2009**
(21) Application number: 06805143.2
(22) Date of filing: 02.11.2006
(51) Int. Cl.: C12N 1/14, A01N 63/04, C12N 15/00, C12N 15/11, A01M 1/00, A01P 7/04, C12R 1/645

(54) **METARHIZIUM ANISOPLIAE VAR. DCJHYIUM AND USES THEREOF**

(71) Applicant: Green Life Laboratory Limited, Hong Kong (CN)
(72) Inventor: HU, Yuanyang, Hubei 430072 (CN); DONG, Changjin, Hubei 435002 (CN); ZHANG, Jiamin, Hubei 430072 (CN); CAO, Xu, Central Hong Kong (CN)
(74) Representative: Sandmann, Wolfgang
(86) International application number: PCT/CN2006/002942
(87) International publication number: WO 2008/052391

(57) **Abstract**

This invention discloses an isolated *Metarhizium anisopliae,* (*M. anisopliae **var.** dcjhyium*), which is referred as Metarhizium anisopliae Lj01. It is identified as a new variant of Metarhizium anisopliae by the morphologic, physiologic, biochemical and molecular biologic analysis (CCTCC No. M206077, GenBank accession number: DQ288247). Also disclosed are the bioinsecticide produced by *M. anisopliae **var.** dcjhyium* (*Metarhizium anisopliae* Lj01.) and this variant in the extermination of pests, such as termites.

## Description

### TECHNICAL FIELD OF THE INVENTION

This invention refers to bio-pesticides, particularly, refers to *Metarhizium anisopliae **var.** dcjhyium* Lj01 (CCTCC deposit number: M206077) and the bio-pesticides comprising said strain, and the method of using them in extermination of insects.

### BACKGROUND OF THE INVENTION

Individual termite is small but may be one of the main pests of the world, which may raise threat and damage in almost all fields of economy, particularly severe to, such as, housing construction, dams of reservoirs, forests and orchards, and communications equipments and facilities, etc. Around the world, with the exception of Antarctica, termites exist in every continent. They have endangered almost 50% of the land area. Especially in tropical and subtropical areas, termite caused damage is more serious and severe. So far, in China, from Taiwan Province to the south-eastern Tibet, from the South China Sea islands to Liaoning province, with the exception of Heilongjiang, Jilin, Inner Mongolia, Ningxia, Qinghai and Xinjiang provinces, the termites live in 25 provinces, municipalities, or autonomous regions, which is nearly 40% of the total national area. It is estimated that the national economic losses caused by termites is about 2 to 2.5 billion yuan per year. This number is particularly high in the area south of the Yangtze River. Meanwhile, the damage of cultural relics and dam of reservoir caused by termites is hard to be directly calculated with economic statistical data. Termite control mainly comprises chemical control and biological control. Using traditional chemical process in termite control may bring in a series of environmental problem. For example, traditional chemical process may contaminate or even destroy the ecological environment. After application of traditional chemical process, termite may recur. Furthermore, traditional chemical process also can kill the native predator of termite (such as birds, spider, ant, mosquito hawk, etc) . Long-term use of chemical pesticide in high dose render the termites generate resistance to the applied chemical insecticide. In this situation, the use not only can not effectively control termites, but also may result in three inextricable puzzles: ① human and livestock may be poisoned because of the environmental contamination and increasing residual toxicity; ② control outlay will be increased because of the increasing dosage concentration and the development of new pesticide; ③ native predator lesion and ecological equilibrium destruction may cause the rampancy or firestorm of the pest, which will result in a vicious circle in nature. These three inextricable puzzles are referred internationally as "3R" ( Residue, Resistance and Resurgence). Comparing with the chemical pesticide, microbial insecticide used for termite control has following merit: ① it is not easy for the termites to generate resistant; ② it is harmless to the vertebrate and human because of high specificity; ③ possess nature epidemic ability; ④ without environmental contamination ; ⑤ it is able to protect the native predator of the pest. Currently, the microbial insecticide used for termites biological control in the world primarily is *Beauveria* and *Metarrhizium anisopliae*, particularly is *Metarrhizium anisopliae*.

*Metarrhizium anisopliae* belong to imperfect fungi, *subphylum metarrhizium anisopliae* genus. *Metarrhizium anisopliae* will reproduce in great quantity in hemocoele after spore germination and invading into the body wall of insect, and thereby kill the insect. *Metarrhizium anisopliae* conidiophores adhere to the body surface of the host. Spore germination generates hypha which invade into body surface, secrete a variety of enzyme, such as chitinase, proteolytic enzyme, lipase, etc., and thereby destroy the body surface and invade into the inside of insect body. The hypha will generate vegetative hypha *in vivo* which can continually ramify. At the same time, a series of "cricoid six peptides toxin " (destruxin A - E ) can be generated during the growth process. Infusing the destruxin into insect will immediately cause paralysis and muscular relaxation of the insect. Besides, the cell membrane, cytoskeleton, morphology of cell nucleus and Ca²⁺ equilibration of the insect will also be destroyed. On the other hand, the invasive hypha is reproduced and diffuse *in vivo* and form a net structure which can invade into various tissue and organ of the termites and thereby destroy the termites' body.

Chinese Patent Application Serious No: 93117629.8, entitled: "The Biological Control of Termites", has disclosed the use of fungi (*Gloeophyllum trabeum*) to tempt termites to contact with *Metarrhizium anisopliae* (*M. anisopliae* ATCC 62176) and thereby kill the termites. However, the used *Metarrhizium anisopliae* (*M. anisopliae* ATCC 62176) has a termites repellent effect. Therefore, it is necessary to use fungi (*Gloeophyllum trabeum*) to cover up the repellent effect.

Stimac (United States Patent Number 4,925,663 (1990)) found that *Beauveria bassiana* can be used to control *Solenepsis*. The mixture of rice, the mycelium and sporesthe can be swallowed by the Solenepsis or moved to the nest.

Stimac et al., (U.S. Patent number 5,683,689) found that the conidiophore of *Beauveria bassiana* grew in rice can be used to control cockroach, carpentry ant and pharaoh ant.

Sugiura, et al., [U.S. Patent No. 5,728,573 (1998)] found that the dormant spores of the germinating fungi and insect-parasitic fungus, such as *brucellosis, Beauveria bassiana*, multi-shaped *Beauveria bassiana, Metarhizium anisopliae* and Wax *sinensis Verticillium* fungi, can be used to kill insects such as termites, cockroaches, ants, pill wood lice, sow bugs, large centipedes, and shield centipedes.

Milner et al., (1997) was granted United States Patent No. 5,595,746 regarding the control of termites. In this patent, *Metarhizium anisopliae* spores were found. Milner et al allege that *Metarhizium anisopliae* spores can be used as a termite expellant in non-pest-infested areas or be used in a termite control method in the infested areas.

As early as the beginning of 1965, the international community has undertaken a great deal of research on using *beauveria bassiana* and *Metarhizium anisopliae* to control termites ( Culliney, T. W, Grace, J. K, 2000. Prospects for the biological control of subterranean termites, with special reference to Coptotermes formosanus. Bull. Entomol. Res, 90, 9-21. Grace, J. K, 1997. Biological control strategies for suppression of termites. J. Agric. Entomol. 14, 281-289 ) . These two fungi have been confirmed to have a good effect on termites control in laboratory, but the effect is very poor in the wild (Hänel, H, Watson, J. A. L, 1983. Preliminary field tests on the use of Metarhizium anisopliae for the control of Nasutitermes exitiosus. Bull. Entomol. Res. 73, 305-313. Milner, R.J, 2003. Application of biological control agents in mound building termites-experiences with Metarhizium in Australia. Sociobiology 41, 419-428 ) . One of the main reasons of poor field result is the great repellency of termites to *Beauveria bassiana* and *Metarhizium anisopliae*; Secondly, they have low toxicity. Third reason is the removal and isolation of the ill or dead individual from the termites community ( Rath, A. C. , 2000. The use of entomopathogenic fungi for control of termites. Biocontrol Sci. Technol. 10, 563-581).

A commercial product comprising *M. anisopliae* as the biological pesticide for termites control had been registered in United States in 1994, but soon was withdrawn for lacking toxicity and low effectiveness in the field test.

In 1997, the Patent Office of the United States granted a patent, which used *M. anisopliae* as biological pesticide for termite control (Milner et al, U.S. Patent No. 5,595,746, 1997). However, this patent also has to face with the lower-toxicity and high-repellency problem ( Wang, C. L., Powell, J. E., 2004. Cellulose bait improves the effectiveness of Metarhizium anisophae as a microbial control of termites. Biological Control. 30, 523-529 ) .

Therefore, there is an urgent need for an effective biological pesticide with highly toxicity and lower repellence to the termites, to control the growing problem of termites, and other harmful pests.

### SUMMARY OF THE INVENTION

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

We found that the *Metarhizium anisopliae var. Dcjhyium* Lj01 (deposit number is CCTCC M206077) is effective for controlling and killing insect pests, and has good entomopathogenic property especially to the termites of *Reticulitrmes, Coptotermes, Odontotermes*, for example, *Coptotermes formosanus* Shiraki, *Reticulitermes chinensis* Snyder, *Odontotermes formesanus* Shiraki. *Metarhizium anisopliae var. dcjhyium* Lj01 is a new variant strain of *Metarhizium anisopliae* which can be cultured in the low-cost medium. The spores generated by *Metarhizium anisopliae var*. *dcjhyium* Lj01 can be used to prepare biological pesticides.

*Metarhizium anisopliae* var. *dcjhyium* Lj01 has been deposited in China Center for Type Culture Collection with the Deposit No.CCTCC M206077. China Center for Type Culture Collection is located in Wuhan University. The deposited *Metarhizium anisopliae var*. *dcjhyium* Lj01 has been proved to be alive.

According to the discovery of the present invention, one purpose of this invention is to isolate a fungal variant with high toxicity, low-repellency or non-repellency to insect pests, from the natural environment.

Another purpose of this invention is to provide biological insecticides with effective insect killing ability both in laboratory and in the wild.

A yet another purpose of this invention is to isolate a fungal variant with high toxicity, low-repellency or non-repellency to termites, from the natural environment.

A yet another purpose of this invention is to provide biological insecticides with effective killing ability to *Reticulitermes chinensis* Snyder and the nest thereof both in laboratory and in the wild.

A yet another purpose of this invention is to provide biological insecticides with effective killing ability to *Coptotermes formosanus* Shiraki and the nest thereof both in laboratory and in the wild.

A yet another purpose of this invention is to provide biological insecticides with effective killing ability to *Odontotermes formesanus* Shiraki and the nest thereof both in laboratory and in the wild.

A yet another purpose of the present invention is to provide a method for killing the pest, such as termites, etc.

### BRIEF DESCRIPTION OF THE DRAWINGS

Figure 1 is a scanning electron microscope photo showing that the spores of the M. anisopliae var.dcjhyium Lj01 distributes in the spore clusters in a fence-like arrangement;
Figure 2 is a scanning electron microscope photo showing that the clusters of spores of *M. anisopliae var.dcjhyium* Lj01 in PDAmedium;
Figure 3 is a scanning electron microscope photo showing that the mucelium of *M. anisopliae var.dcjhyium* Lj01 grown in termites;
Figure 4 is a scanning electron microscope photo showing that the clusters of spores of *M. anisopliae var dcjhyium* Lj01 grown in the head of termites;
Figure 5 is the esterase isoenzyme electrophoretic pattern of *M. anisopliae* var. *dcjhyium* Lj01 (CCTCC M206077). Lane 1=AB027337, lane 2 = AB099941, Lane 3=AF280631, Lane 4=AB099510, Lane 5= CCTCC M206077;
Figure 6 shows that part sequence of Ribosome 18S r DNA reflects the different sites (bold-faced) between *M. anisopliae* var. *dcjhyium* Lj01 (CCTCC M206077) and several other variants (such as AB099510, AB099941, AF280631, and AB027337);

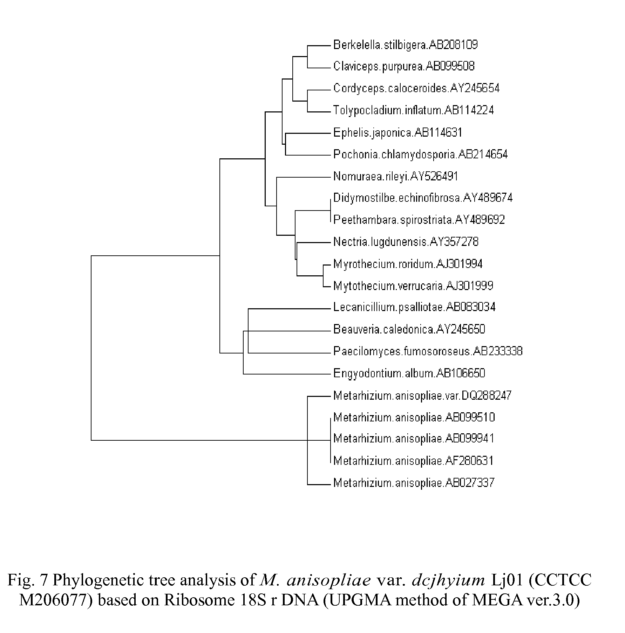

Figure 7 shows the Phylogenetic tree analysis of *M. anisopliae* var. *dcjhyium* Lj01 (CCTCC M206077) based on Ribosome 18S r DNA (UPGMA method of MEGA ver.3.0);
Figure 8 is a Schematic drawing of a termite insecticide-kit;
Figure 9 is a schematic drawing of another termite insecticide-kit with different design;
Figure 10 shows the dose effect of *M. anisopliae* var. *dcjhyium* Lj01 (CCTCC M206077) to termites.

The other characteristic and advantages of the present invention will be apparent from the following detailed description and claims in combination of those figures.

### DETAILED DESCRIPTION OF THE INVENTION

The inventors of the present application have isolated a new strain of insecticidal fungi from the body of dead termites in the nest located in Wuhan City, Hubei Province, Wuhan University Luojia Hill field, and produced its pure cultures.

In one embodiment of the present invention, *M. anisopliae* var. *dcjhyium* Lj01 (CCTCC M206077) has been isolated and cultured to provide a substantially homogeneous culture that is pathogenic to insect pests. Isolation, identification and cultivation of *Metarhizium anisopliae* var. *dcjhyium* Lj01

### 1. Isolation and cultivation of Metarhizium anisopliae var. dcjhyium Lj01

The inventors has successfully isolated a new insecticidal fungus and produced their pure cultures from dead body of termite found in the nest located in the Luojia Hill field in Wuhan City, Hubei Province, Wuhan University. The isolated fungus was cultured in the improved solid PDA medium (Potato 200g, sugar 20g, peptone 10g, agar 20g, water 1000ml. 121 °C, sterilized under 1 atmospheres for 1hr), under a constant temperature of 28 °C.

No creative work is needed for the skilled person in the art to select other mediums suitable for *Metarhizium anisopliae* var. *dcjhyium* Lj01.

### 2. Identification of Metarhizium anisopliae var. dcjhyium Lj01

1) Toxicity Comparison of *Metarhizium anisopliae* var. *dcjhyium* Lj01
Sample strains: Five variants of Metarhizium *anisopliae* (CCTCC M206077, AB099510, AB099941, AF280631and AB027337) were cultured in an improved PDA medium for 10 days under the constant temperature of 28 °C. Spores were diluted to 3×10⁷ - 3×10⁸spore/ml using 0.05% Tween-80.
Sample termites: *O. formosanus* Shiraki
A piece of filter paper was put at the bottom of a beaker and moistened with cotton balls.50 *O. formosanus* Shiraki were put into the beaker. Then, in the test group, 1ml 3×10⁷ - 3×10⁸ spore/ml *Metarhizium anisopliae var. dcjhyium* Lj01 were put into the beaker, in the control, 1 ml 0.05% Tween 80 were introduced. Every test was repeated for 5 times. The results were shown in following Table 1:

**Table 1 The toxic comparison of Metarhizium anisopliae var. dcjhyium( CCTCC M206077 ) vs. O. formosanus Shiraki**

| Treatment | Termite mortality (%) | | | | |
|---|---|---|---|---|---|
| | 3 days | 6days | 9days | 12 days | 15 days |
| 0.05%Tween-80 (Control) | 0.0 | 1.0 | 1.5 | 2.8 | 5.0 |

| 3×10⁷ spore/ml | | | | | |
|---|---|---|---|---|---|
| CCTCC M206077 | 76.8 | 100.0 | 100.0 | 100.0 | 100.0 |
| AB099510 | 3.2 | 15.5 | 41.1 | 73.3 | 98.6 |
| AB099941 | 5.7 | 20.0 | 56.7 | 94.6 | 100.0 |
| AF280631 | 2.3 | 17.6 | 37.2 | 69.2 | 95.4 |
| AB027337 | 2.0 | 13.4 | 39.5 | 67.8 | 93.2 |

| 3×10⁸ spore /ml | | | | | |
|---|---|---|---|---|---|
| CCTCC M206077 | 100.0 | 100.0 | 100.0 | 100.0 | 100.0 |
| AB099510 | 6.0 | 21.1 | 53.5 | 86.4 | 100.0 |
| AB099941 | 8.3 | 25.4 | 75.3 | 100.0 | 100.0 |
| AF280631 | 5.7 | 18.6 | 59.2 | 97.1 | 100.0 |
| AB027337 | 6.5 | 23.2 | 57.7 | 84.2 | 100.0 |

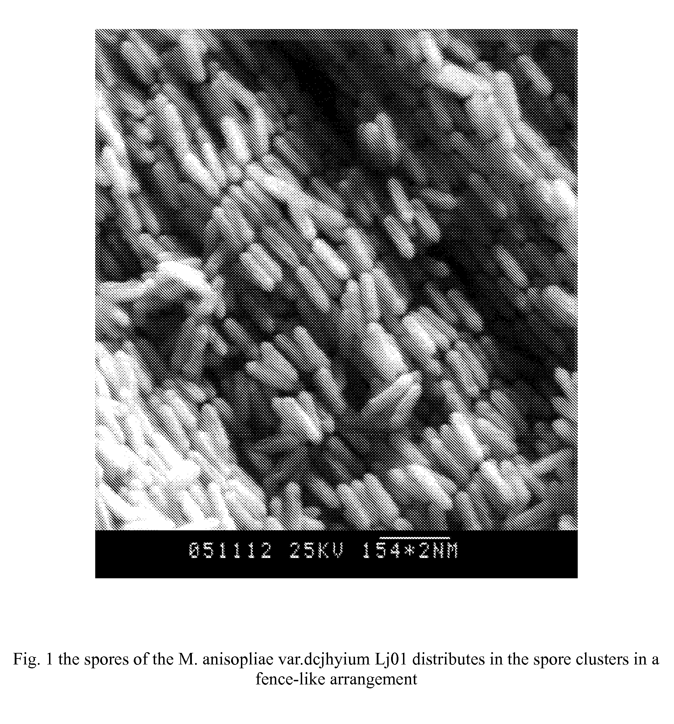

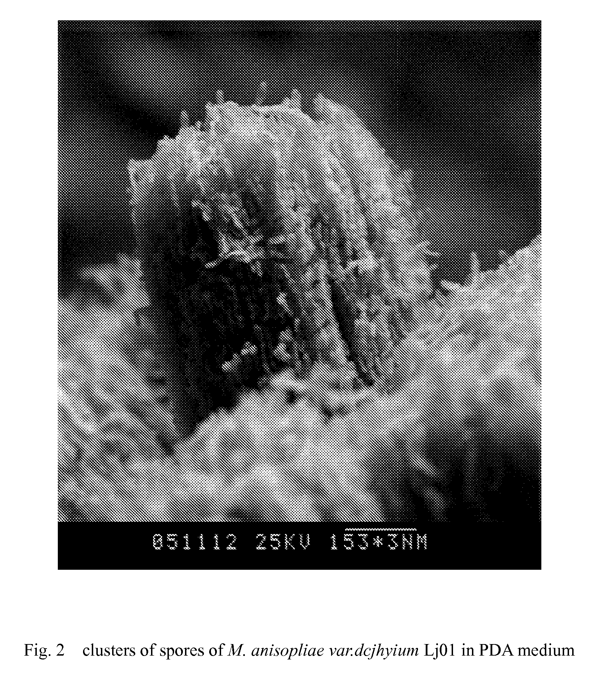

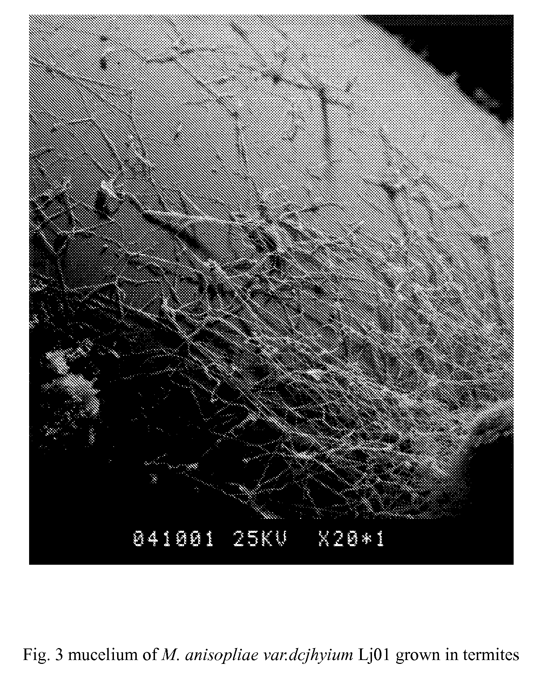

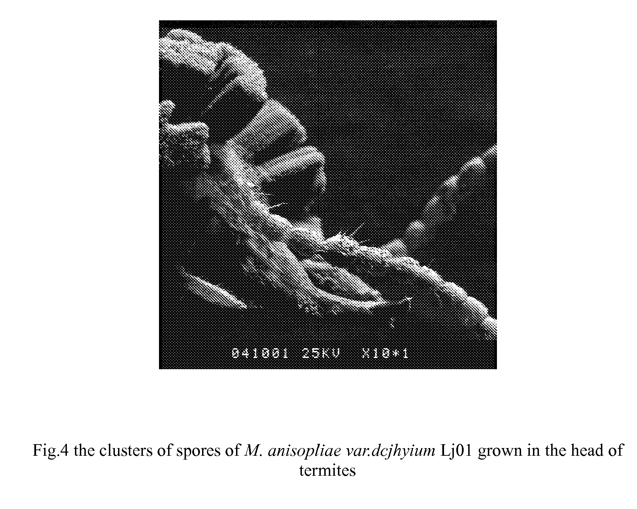

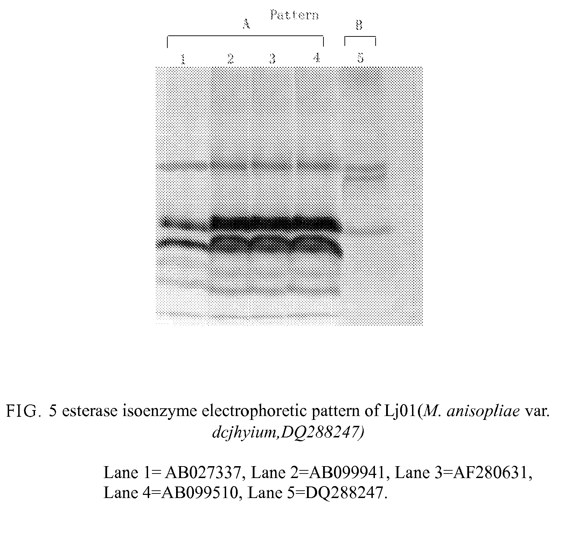

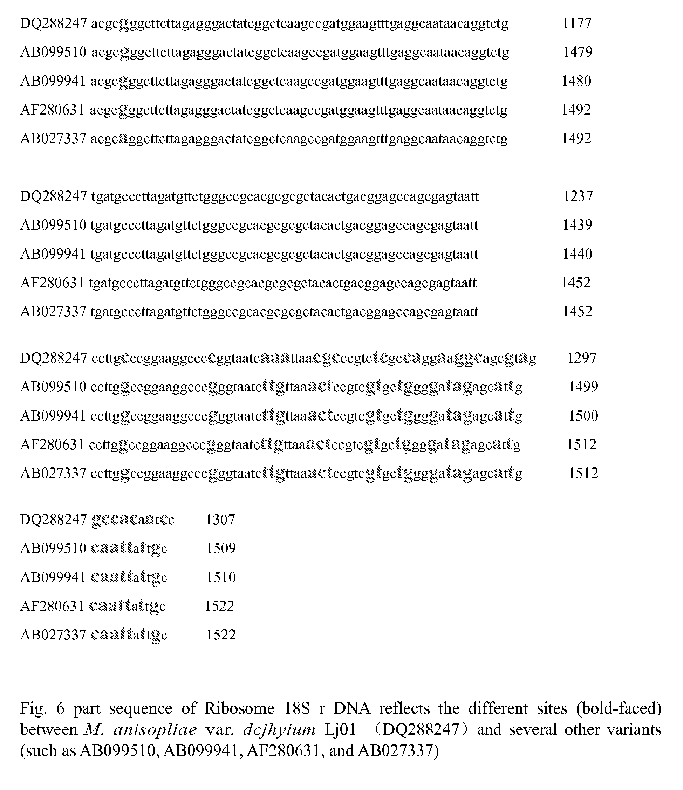

The results show that, when *Metarhizium anisopliae var. dcjhyium* Lj01 (CCTCC M206077) with the concentration of 3 × 10⁸ spores/ml is used, the mortality of *O. formosanus* Shiraki in the third day was 100%. However, when treated by the other four variants of *Metarhizium anisopliae* (AB099510, AB099941, AF280631 and AB027337) under the same concentration, the mortality of *O. formosanus* Shiraki was less than 10% in the third day. It may take 12-15 days to achieve 100% mortality. Therefore, comparing with the other variants of *Metarhizium anisopliae, Metarhizium anisopliae var. dcjhyium* Lj01 (CCTCC M206077) show the strongest toxicity to *O. formosanus* Shiraki.
2) After three days from inoculation of *Metarhizium anisopliae var. dcjhyium* Lj01 (CCTCC M206077), a large number of white colonies, which were villous or floccular, were generated. After 9 days of inoculation, green spores were generated and then turn to black. The white colonies and spores present simultaneously with low growth rate. Observed with optical microscopic, the spores are in a long cylindrical shape. Scanning electron microscope shows that the cylindrical spores grow in cluster and arrange in the form of the palisade (Fig. 1-2). For the termites inoculated with cultures, mycelium and spores grew in the body of the dead termites, and the long cylindrical spores arranged in clusters on the body of the dead termites (fig 3-4).
3) *Metarhizium anisopliae var. dcjhyium* Lj01 esterase isozyme analysis
5 variants of *Metarhizium anisopliae* (CCTCC M206077, AB099510, AB099941, AF280631 and AB027337) were inoculated into 100 ml sterilized medium (Sugar 10g, peptone 2.5g, yeast extract 5g, water 1000ml), shaking cultured at 180rpm/min28 °C for 60h, filtered to obtain mycelium. Adding 1ml 0.1M TBE (Tris-H3BD4-EDTA) extracts to 1g mycelium, freezing the mixture at -20°C for 24 h, then rubbing and centrifugation (4000r/min, 20min) for the supernatant, and store the supernatant in refrigerator. Conducting vertical flat-panel electrophoresis with polyacrylamide gel (3% concentrated gel, 7% separated gel, electrode buffer Tris-Gly, pH 8.9. 50 ml sample, use bromophenol blue as electrophoresis indicator, with regulators: 120v 0.5h, 175v 2.5h). Stained with fast blue B salt, the result of this electrophoresis is shown in Figure 5. Seen from Figure 5, all of the 5 variants of *Metarhizium anisopliae* show esterase isozyme spectrum (Isozyme is the product of sub-expression of gene, which can be used as a biochemical indicator in genetics. Esterase isozyme can be used to conduct analyses for fungi relationship and gene localization). Comparing with the other 4 variants of *Metarhizium anisopliae* (AB099510, AB099941, AF280631and AB027337), *Metarhizium anisopliae var. dcjhyium* Lj01 (CCTCC M206077) shows a distinct esterase isozyme spectrum. In other words, the spectrum of *Metarhizium anisopliae var. dcjhyium* Lj01 shows 3 loci (Rf are 0.4490, 0.5198 and 0.8367 respectively), while the spectrums of the other four show 6 loci and they only share one common locus (Rf is 0.4490).
4) The genomic DNA of *Metarhizium anisopliae var. dcjhyium* Lj01 is extracted. 18S rDNA is amplified using Taq DNA polymerase and primers: 5'-CCAATGCCCTTCGGGGCTCA-3' and 5'-CCACCTCTCTGGGCCAGTCC -3'. 50 µl PCR reactive system contains: 10×buffer 5µl, 25mmol/L MgCl₂ 3µL, 2mmol/L dNTP 5µL, 10µmol/L primer R 2.5µL, 10µmol/L primer L 2.5µL, 5U/µL Taq E 0.5µL, dd H₂O 26.5µL and DNA template 5 µL (-10 ng). At first round, denaturalization is conducted at 94°C for 3 min, in the other round, denaturalization is conducted at 94°C for 1 min. Annealing at 55°C for 1min, amplifying at 72°C for 1min, and repeated for 35 rounds. In the last round, the amplification step is conducted at 72°C for 10min. Subsequently, amplifying products of 18S rDNA are cloned and sequenced (1400bp). Using the high conservative 18S rDNA sequence of *Metarhizium anisopliae var. dcjhyium* Lj01 (CCTCC M206077) which are input into GenBank to conduct the BLAST alignment, the result shows that the sequence of *Metarhizium anisopliae var. dcjhyium* Lj01 (CCTCC M206077) is 99% identical with those of the other four variants of *Metarhizium anisopliae* (AB099510, AB099941, AF280631and AB027337). 18S rDNA sequence alignment shows that the difference between *Metarhizium anisopliae var. dcjhyium* Lj01 and the other four variants lies in the replacement of 26 base pairs: four g→c, two c→g, five t→a (transfer) and four g→a, two a→g, one g→t, one t→g, three a→c and four t→c (transversion; Fig.6) .
5) 18S rDNA sequence of *Metarhizium anisopliae var. dcjhyium* Lj01 (CCTCC M206077) is analyzed using NCBI Blast Program and then using UPGMA method of MEGA ver. 3.0 to conduct the phylogenetic tree comparison (Fig, 7). *Metarhizium anisopliae var. dcjhyium* Lj01 (CCTCC M206077) form a monophyly with the other four variants of *Metarhizium anisopliae* (AB099510, AB099941, AF280631 and AB027337), which is 99% identical with each other and constitutes the species of *Metarhizium anisopliae*.

Another embodiment of this invention refers to a biological pesticide comprising *Metarhizium anisopliae var. dcjhyium* Lj01. All of the fungi, spores, conidium, mycelium of *Metarhizium anisopliae var. dcjhyium* Lj01 can be used as the active ingredients of the biological pesticide. The preferred is the fungi, spores and conidium of *Metarhizium anisopliae var. dcjhyium* Lj01. The more preferred is the spores thereof, which possess good stability for storage, transport and use.

Active ingredients of the biological pesticide of the present invention can be any or all of the fungi, spores, conidium, mycelium of *Metarhizium anisopliae var. dcjhyium* Lj01. The biological pesticide of the present invention possesses less, if there is any, or no repellency to insect pest, particularly to termite.

In a yet another embodiment of the present invention, the biological pesticide is a termites-killing agent comprising the fungi, spores, conidium, mycelium or any mixture thereof of *Metarhizium anisopliae var. dcjhyium* Lj01 as the active ingredient, preferably, comprising the fungi and the spores thereof, more preferably, comprising the spores. The termites-killing agent possesses small or even no phobotaxis to termite. The termites-killing agent of the present invention is effective in killing various termites, for example, but not limit to: *Coptotermes formosanus* Shiraki, *Reticulitermes chinensis* Snyder, *Odontotermes formosanus* Shiraki, *Macrotermes barneyi* Light, *Cryptotermes domesticus* Haviland, *Kalotermes flavicollis* Fabr., *Cryptotermes brevis* Walker, *Reticulitermes santonensis* Feytaud, *Heterotermes indicola* Wasmann, *Reticulitermes flavipes* Kollar, *Reticulitermes Hesperus* Banks, *Zootermopsis angusticollis* Hagen, *Nasutitermes moratus* Silvestri, *Coptotermes curvignathus* Holmgren, *Incisitermes minor* Hagen, *Reticulitermes virginicus* Banks, *Macrotermes michaelseni* Sjostedt, Macrotermes bellicosus Smeathman, etc.

The bio-pesticides of the present invention are effective to insects. In particular, the fungi, spores, conidium, mycelium and cultures of *Metarhizium anisopliae var. dcjhyium* Lj01 or any mixture thereof possess strong etiologic activity to insects. Without limitation by any mechanisms, the *Metarhizium anisopliae var. dcjhyium* Lj01 of the present invention is a parasitic fungus of various insects. The *Metarhizium anisopliae var. dcjhyium* Lj01 possesses contacting and stomach toxicities, which can be induced into insects by surface contact or eating. The fungi reproduce in the insect and consume the nutrition, mechanically penetrate the pest, release toxin and spread in the pest population to kill the pests.

**Mode of Infection:** *Metarhizium anisopliae var. dcjhyium* Lj01 attach to the epidermal of the host at first. Once growing, *Metarhizium anisopliae var. dcjhyium* Lj01 will infect the host by producing infective hypha and thereby kill the host. During this process, *Metarhizium anisopliae var. dcjhyium* Lj01 form a special structure, such as appressoria etc., and at the same time excrete various relative enzymes, such as chitinase, protease, lipase, etc., destroy the epidermal and infect into the body of the host. Besides, *Metarhizium anisopliae var. dcjhyium* Lj01 produce the hypha, which may continually branch.

Appressoria is the conidium attached on the skin of the host, which may form the germ tube after germination. The germ tube has good directional property to the epidermal. A series of precise recognition structures such as appressoria, penetration peg and infection hypha are needed for penetrating the epidermal of the insect. These structures effectively focus the physical (mechanical) and dissolution abilities of the fungi to a small range of the skin, which may help to infect into the insect. The appressoria contains a large number of mitochondria, Golgi apparatus, endoplasmic reticulum and ribosomes, and is extremely effective in synthesis and excretion of hydrolase.

**Enzyme**: the epidermal of termite is mainly made of protein, chitin and lipid, wherein the main components are proteins, chitin microfibers, which are the initial invasion sites of *Metarhizium anisopliae var. dcjhyium* Lj01. One strategy of *Metarhizium anisopliae* for degrading epidermal is to produce various extracellular enzymes. The epidermal of termite is degraded by corresponding enzyme (the degrading enzyme of protein, chitin and lipid) produced by hyphae during the invasion, and thereby make it easier for further invasion.At the same time, this degradation provides nutrients to mycelia for their further growth.

**Toxin synthesis and spreading of mycelium:** Base on current view, fungal toxins function by interfering the immune system of host cells. *Metarhizium anisopliae* may produce a series of "six peptide ring toxin"(destruxin) during growth. Once injected into termites, these toxins immediately cause paralysis and muscle relaxation, damage of cell membrane, cytoskeleton, karyon shape and the Ca^{2 +} balance, etc. At the same time, the mycelium that has invaded insect will reproduce in termites and spread to form a net structure, which invade every tissue or organs of the termites and thereby destroy the entire termite.

Except for abovementioned characteristics, *Manisopliae var. dcjhyium* Lj01 further possesses the following advantages: ① strong toxicity; ② small repellency to termites; ③ other advantages such as rapidly killing and effectively controlling the termites. *M.anisopliae var. dcjhyium* Lj01 is able to adapt to the living conditions (nutrition, temperature, humidity, CO₂ concentration, etc.) of termite nests and has the ability of infection on termites, and eventually infect and kill each individual termite to reach the purpose of extermination of the entire termite colony within very short time (30-50 days).

Another embodiment of this invention refers to a biological pesticide comprising *Metarhizium anisopliae var. dcjhyium* Lj01 or spores, conidium, mycelium or any mixture thereof. Besides, this pesticide further comprises insect pheromones.

Termite tracing pheromone refers to chemicals excreted by the termite for interrelation and transferring information among the colony of termite. The pheromone is needed when the termite defending, nesting, looking for food and individual identifying, etc. Pheromone analog refers to the chemical that is obtained (such as synthesized) from the other source except termites and possesses similar activities with termite tracing pheromone. There are many advantages for using pheromone analog to tempt and control termite, such as high efficiency, innocuity, no pollution and no hurt to the beneficial insect. Consequently, pheromone analog possesses a prospect of wide application. Termite lives about 2 - 5m underneath. The synthesis of artificial termitic pheromone assists the function of *Meterhizium anisopliae* ***var*.** *dcjhyium* Lj01 in controlling termites. We have completed chemical synthesis of (Z,Z) - 3,6 - codlemone - 1, which is the tracing pheromone analogs of *Odontotermes formesanus* **Shiraki.**

With the presence of lithium amide, using propiolic alcohol and bromine pentane as raw material, 2-octyne alcohol - 1 is obtained. The result conducts bromization with the presence of phosphorus tribromide. After bromization, the products further react with 3-butine alcohol-1 in the presence of catalyzer such as cuprous ion and bromethyl magnesium to produce 3,6-dodecadiyne diyne alcohol. Finally, selected cis-hydrogenation is conducted by Lindlar catalyzer with the yield of 42%. The final product is a kind of colorless oil with GC purity of 94.3%. This synthesis procedure is shown as follow:

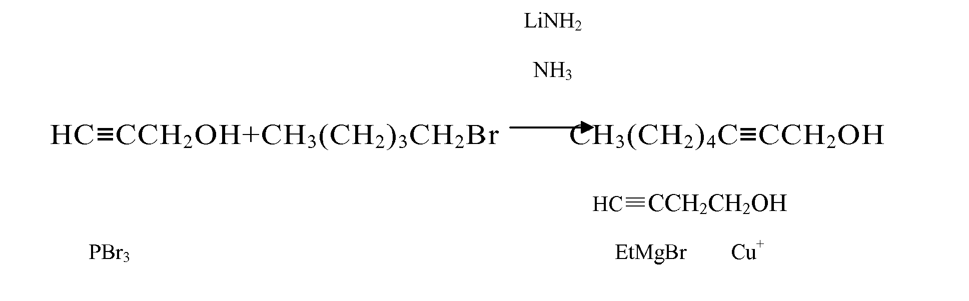

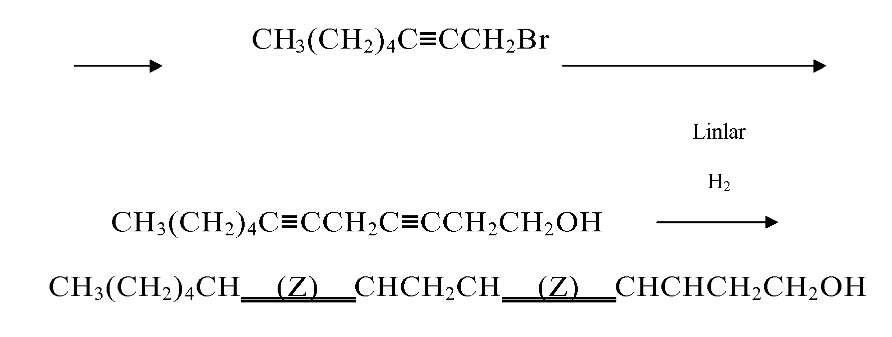

The bioactivities of artificially synthesized termite pheromone are tested by Figure - Y and Student test. The results show that, under the optimum concentration, no significant difference is found in the response of *Odontotermes formesanus* Shiraki to either natural pheromone extract or the synthesized pheromone. Consequently, the synthesized (Z,Z) - 3,6 - Codlemone - 1 possesses desirable activities.

Another embodiment of the present invention refers to a bio-pesticides comprising *Meterhizium anisopliae var. dcjhyium* Lj01, insect pheromone, and optionally insect bait.

The insect bait used in the bio-pesticides of the present invention includes various of food attractant comprising, but not limited to, cornmeal, pollen pini, bagasse, bracken, sucrose, protein, yeast, treacle, pine rottenwood powder, paulownia rottenwood powder, blackfungus powder, sodium glutamate, L-leucine, aspartate, vitamin b, dextrin, caramel, etc. Wherein, cornmeal, pollen pini, bagasse, blackfungus powder and sucrose are more effective. Food attractant can be used as an complement to the effect of tracing pheromone. The skilled person in the art can select the suitable bait according to the specific condition.

The bio-pesticides of the present invention further comprise various adjuvants, such as various stuffing include , but not limited to pine saw powder, natural protein of animals or plants, starch, dextrin, sugar powder, lactose, microcrystalline cellulose, soluble starch, inorganic salt, mannitol, etc. Wherein, pine saw powder , microcrystalline cellulose, natural animal or vegetable protein are more effective. The skilled person in the art can select the suitable stuffing according to the specific condition.

Furthermore, other suitable adjuvants used in the bio-pesticides of the present invention further include various bonder, such as, including but not limited to fibre powder, lecithin or cornstarch, starch slurry, sodium carboxymethylcellulose (CMC-Na), hydroxypropyl cellulose (HPC) , methyl cellulose (MC) , Ethocel (EC), hydroxypropyl methylcellulose (HPMC) , gelatin, Polyvinyl pyrrolidone (PVP) etc. Wherein, sodium carboxymethylcellulose, lecithin and cornstarch are more effective. The skilled person in the art can select the suitable bonder according to the specific condition.

It has been verified by virulence test inside laboratory and termite control test in field that the termite pesticides of the present invention comprising *M. anisopliae var. dcjhyium* Lj01 as the active ingredient, insect pheromone as the attractant , complementing with food attractant can be used in the biological control of all termites with good effect in controlling and killing termite's species.

For example, the termites that can be killed by the pesticides of the present invention include, but not limited to *Coptotermes formosanus* Shiraki, *reticulitermes chinensis* snyder, *odontotermes formosanus* shiraki, *macrotermes barneyi* light, *crgptotermes domesticus* haviland, *kalotermes flavicollis* fabr., *Cryptotermes brevis* Walker, *reticulitermes santonensis* feytaud, *heterotermes indicola* wasmann, *reticulitermes flavipes* kollar, *reticulitermes hesperus* banks, *zootermopsis angusticollis* hagen, *nasutitermes moratus* silvestri, *coptotermes curvignathus* holmgren, *incisitermes minor* hagen, *reticulitermes virginicus* banks, *macrotermes michaelseni* sjostedt *and* macrotermes bellicosus smeathm, etc.

Surprisingly, the bio-pesticides of the present invention also possess strong pathogenicity to many other pest species. For example, except different kinds of termite, the bio-pesticides of the present invention also can be used in the biological control of other pests such as *Solenopsis invicta,* carpenter ants, pharoach ants, locust, *dendrolimus* and cockroach.

These pests will be controlled and killed by employing the bio-pesticides of the present invention near their nest or their path.

In a yet another embodiment of the present invention, the bio-pesticides of the present invention comprising *M. anisopliae var. dcjhyiu*m Lj01 as the active ingredient can be used in various formulations.

The common formulations of bio-pesticides comprising *M. anisopliae var. dcjhyium* Lj01 include, but not limited to powder, oil suspension, slug (disinsection kit), microcapsules, oil base fog, powder casted by rocket, ultraviolet protecting agent, water suspension, nanoagent, etc. Wherein, the slug (disinsection kit), powder casted by rocket, and powder are more effective. The skilled person in the art can select the suitable formulation according to the specific condition.
①powder: Spore powder will be produced by adsorbing the *M. anisopliae var. dcjhyium* Lj01 spore by suitable carrier.
② oil suspension: The oil suspension can be produced by suspending *M. anisopliae var. dcjhyium* Lj01 spore in the mineral oil and adding suitable amount of potentiator (MgCl₂ MgSO₄ and Na₂CO₃).
③slug (disinsection kit): The slug can be produced using *M. anisopliae var. dcjhyium* spore as active ingredient, insect pheromone as the attractant and an amount of food attractant, stuffing and bonder. The disinsection kit can be produced by putting the produced slug into the suitable device.
④microcapsules: Microcapsule formulation refers to sphere with the size of several to hundred of micrometer, wherein the *M. anisopliae var. dcjhyium* Lj01 spore is capsulated in the microcapsules (such as starch, inorganic salt, etc). The release rate of the medicament can be controlled by altering the thickness of the microcapsules and pore size.
⑤oil based fog: Being suspended in NO.0 diesel oil and atomized by impulse type fog machine, *M. anisopliae var. dcjhyium* Lj01 spore still hold their toxicity and activity after sparging from the muzzle, thereby may kill the Dendrolimus directly.
⑥ powder casted by rocket: After formulated, 600g±50g of *M. anisopliae var. dcjhyium* Lj01 spore was enclosed in the rocket warhead, which will up to the air and blast to disperse the spore powder over the target leaf.
⑦ultraviolet protecting agent: Addition of lignin and sucrose solution into *M. anisopliae var. dcjhyium* Lj01 spore powder can prevent the powder from ultraviolet (ultraviolet radiation from sunshine is the primacy factor which depress the effect of *M. anisopliae var. dcjhyium* Lj01 spore powder) and extend the useful-life of the powder.
⑧water suspension: *M. anisopliae var. dcjhyium* Lj01 spore powder was added to 0.05% Tween80 to produce the water suspension.
⑨nanoagent: The nanoagent can be produced by adding *M. anisopliae var. dcjhyium* Lj01 spore powder to suitable carrier (such as starch, mineral salt etc.) through nanometer technology.

Another embodiment of the present invention provides a method of controlling and killing pest using the bio-pesticides of the present invention comprising *M. anisopliae var. dcjhyium* Lj01 spore.

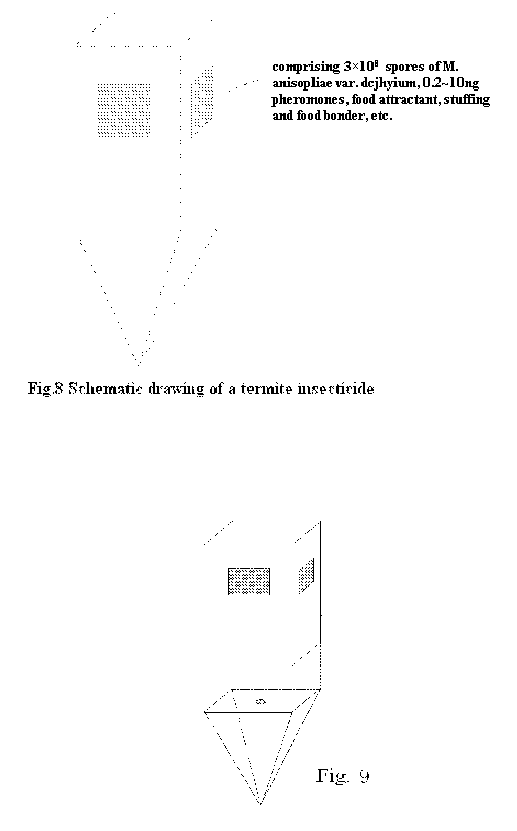

The using method of the bio-pesticides comprising *M. anisopliae var. dcjhyium* Lj01 spore include , but not limited to sprinkling, killing by the kit, formicary directly perfusion, atomizing using impulse fog machine, or sprinkling by plane or rocket, etc. The present invention also provides a convenient device that is useful to the indoor or outdoor termite. This device is shown in Fig. 8. This device is a kind of kit with the monolithic construction as shown in Fig 8. It is basically a long cylinder with an acute terminal apt to be embedded into the underground and another terminal that is not in acute shape and is empty inside to carry out the bio-pesticides of the present invention. In the other hand, the acute terminal also can be designed to have a hollow part for the bio-pesticides of the present invention.

In the other embodiment of the present invention, as shown in Fig.9, the disinsection kit comprises two parts. One possesses the acute terminal and the other does not. The non-acute part is empty inside. Therefore, it is easy to carry out the bio-pesticides of the present invention. said acute part and non-acute part are bound to each other by a locking apparatus. If the kid is proposed to be used indoor, only non-acute part is need. The non-acute part may further comprise some auxiliary device such as double paste to adhere the part at the selected position. If the kid is proposed to be used outdoor, both parts of the kit shall be fixed together by the locking apparatus and conveniently embedded into the underground.

It is understood by the skilled person in the art that the present invention contains various modifications or alterations without depart from the spirit of the invention. Those modifications and alternations are within the scopes limited by the claims of this application.

As mentioned above, the bio-pesticides of the present invention also can kill the locust, dendrolimus and cockroach, etc. Especially to locust and dendrolimus, bio-pesticides will be more effective if sprinkling by plane or rocket.

The following examples will further describe the present invention. However, none of them shall be deemed to be the limit of scopes of the present invention that shall be defined by the claims.

### EXAMPLES:

### Example 1:

Sample strains: *Metarhizium anisopliae* var. *dcjhyium* Lj01 was cultured in the improved PDA medium (Potato 200g, sugar 20g, peptone 10g, agar 20g, water 1000ml. 121 °C, sterilized under 1 atmospheres for 30min)for 10 days under the constant temperature of 28 °C. The spore was diluted to 3×10⁷ - 3×10⁸spore/ml using 0.05% Tween-80.

Sample termites: *O. formosanus* Shiraki

tested pheromone : artificially synthesized tracing pheromone analogs of O. formosanus Shiraki ( z,z) -3,6 - codlemone-1 ( DDE-OH) with the concentration of 0.2∼10ng / ml. a piece of filter paper was put at the bottom of a beaker and moistened with cotton balls. 100 Sample termites, DDE-OH, *Metarhizium anisopliae* var. *dcjhyium* Lj01 spore and 0.05% Tween 80 were mixed together and the final concentration of pheromone analogs is 0.2-100 ng/ml. 1ml 3×10⁷ - 3× 10⁸ spore/ml *Metarhizium anisopliae var. dcjhyium* Lj01 (with DDE-OH) were put into the beaker, in the control, 1 ml 0.05% Tween 80 were put into the control group. Every test was repeated for 5 times. The results were shown in Table 2:

**Table 2: The toxic comparison of Metarhizium anisopliae var. dcjhyium Lj01 vs. O. formosanus Shiraki**

| Treatment | | Termite mortality | | | |
|---|---|---|---|---|---|
| Lj-01 | DDE-OH(ng/ml) | 1 days | 2 days | 3 days | 4 days |
| Control group ( 0.05%Tween-80 ) | 0 | 0.0 | 1.0 | 3.0 | 3.0 |
| | 0.2 | 0.0 | 1.0 | 3.0 | 3.0 |
| | 2 | 0.0 | 1.0 | 3.0 | 3.0 |
| | 4 | 0.0 | 1.0 | 3.0 | 3.0 |
| | 6 | 0.0 | 1.0 | 3.0 | 3.0 |
| | 8 | 0.0 | 1.0 | 3.0 | 3.0 |
| | 10 | 0.0 | 1.0 | 3.0 | 3.0 |
| | 20 | 0.0 | 1.0 | 3.0 | 3.0 |
| | 50 | 0.0 | 1.0 | 3.0 | 3.0 |
| | 100 | 0.0 | 1.0 | 3.0 | 3.0 |
| 3×10⁷spore/ml | 0 | 23.6 | 51.4 | 76.8 | 100.0 |
| | 0.2 | 24.8 | 53.8 | 79.9 | 100.0 |
| | 2 | 26.9 | 59.2 | 83.6 | 100.0 |
| | 4 | 30.5 | 66.7 | 89.5 | 100.0 |
| | 6 | 38.0 | 79.5 | 96.3 | 100.0 |
| | 8 | 36.6 | 72.3 | 91.1 | 100.0 |
| | 10 | 29.3 | 67.4 | 80.5 | 100.0 |
| | 20 | 18.4 | 46.6 | 63.3 | 92.7 |
| | 50 | 13.2 | 25.7 | 47.5 | 72.6 |
| | 100 | 8.9 | 17.5 | 32.6 | 49.3 |
| 3×10⁸spore/ml | 0 | 38.8 | 93.3 | 100.0 | |
| | 0.2 | 39.9 | 95.0 | 100.0 | |
| | 2 | 45.3 | 98.8 | 100.0 | |
| | 4 | 56.0 | 100.0 | 100.0 | |
| | 6 | 63.7 | 100.0 | 100.0 | |
| | 8 | 57.3 | 100.0 | 100.0 | |
| | 10 | 52.1 | 96.5 | 100.0 | |
| | 20 | 31.5 | 66.7 | 89.8 | 100.0 |
| | 50 | 20.7 | 48.6 | 71.2 | 87.7 |
| | 100 | 13.3 | 27.4 | 43.7 | 62.0 |

### ① Toxicity test in the laboratory

The results show that, DDE-OH in concentration of 6ng/ml possess the best effect on alluring *O. formosanus* Shiraki. When *Metarhizium anisopliae var. dcjhyium* Lj01 was in the concentration of 3 × 10⁸ spores / ml, the best killing effect will be achieved. When DDE-OH was in concentration of 6ng/ml and *Metarhizium anisopliae var. dcjhyium* Lj01 was in the concentration of 3×10⁸ spores / ml, the mortality of *O. formosanus* Shiraki on the first day will achieve 63.7% and on the second day will achieve 100%.

### ② Outdoor Toxicity test

Selecting 3 formicaries of *O. formosanus* Shiraki in the field and embedding the disinsection kit of the present invention around every formicary or every 1-2 m on the pathway of the termites. Every kit contains 3×10⁸ *Metarhizium anisopliae var. dcjhyium* Lj01 spore, 6 ng pheromone DDE - OH, food attractant, stuffing and food bonder. After six weeks, all the termites in 3 formicaries are killed. This result shows that, pheromone DDE - OH and food attractant cooperate with each other to allure the ergates of *O. formosanus* Shiraki go out for food. These ergates will return with *Metarhizium anisopliae var. dcjhyium* Lj01 spore on their surface or mouth. Therefore, the *Metarhizium anisopliae var. dcjhyium* Lj01 spore will be carried in and then grow to fill up the entire formicary. As a result *Metarhizium anisopliae var. dcjhyium* Lj01 spore will kill all the termites in the formicary by inter-infecting between individual termites.

### Example 2:

The effective dosage of *Metarhizium anisopliae* var. *dcjhyium* Lj01 *in killing termites*

Sample strains: *Metarhizium anisopliae* var. *dcjhyium* Lj01 was cultured in the improved PDA medium for 10 days under the constant temperature of 28 °C. The spore was diluted to 3×10² - 3×10¹³spore/ml using 0.05% Tween-80.

Sample termites: *O. formosanus* Shiraki, *Coptotermes formosanus* Shiraki and *Reticulitermes chinensis* Snyder.

There are 12 glass beakers for every kind of termites. A piece of filter paper was put at the bottom of the beaker and moistened with cotton balls.100 Sample termites were put int o the beaker and adding to 1ml suspension with the concentration of 3× 10² - 3×10¹³spore/ml, respectively. Each test shall be repeated for 3 times. The time required for half of termites dead shall be recorded. The results were shown in Table 3:

**Table 3: The dosage effect of Metarhizium anisopliae var. dcjhyium Lj01 to termites**

| Spore concentration (spore/ml) | LT₅₀ ( h ) | | |
|---|---|---|---|
| | **O*. formosanus*** | ***C. formosanus*** | *R. **chinensis*** |
| 3×10² | 178.0 | 189.4 | 216.6 |
| 3×10⁴ | 89.7 | 75.5 | 93.0 |
| 3×10⁶ | 64.5 | 49.6 | 69.3 |
| 3×10⁷ | 46.8 | 28.1 | 49.2 |
| 3×10⁸ | 40.0 | 20.9 | 33.4 |
| 3×10⁹ | 40.0 | 20.7 | 33.3 |
| 3×10¹¹ | 40.0 | 20.8 | 33.5 |
| 3×10¹³ | 39.9 | 21.0 | 33.4 |

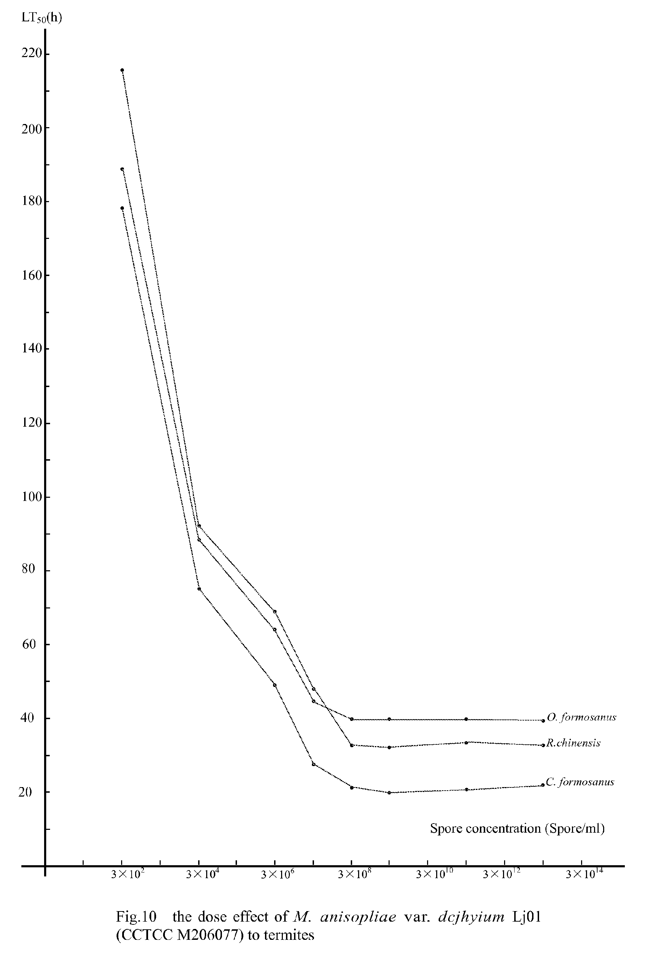

Fig.10 reflects the dosage effect of *Metarhizium anisopliae* var. *dcjhyium* Lj01 to termites. Seen from Fig.10, 3×10⁸ spore/ml is the optimum concentration of *Metarhizium anisopliae* var. *dcjhyium* Lj01 for killing termites.

### Example 3:

Sample strains: *Metarhizium anisopliae* var. *dcjhyium* Lj01 was cultured in the improved PDA medium for 10 days under the constant temperature of 28 °C. The spore was diluted to 3×10⁷ - 3×10⁸spore/ml using 0.05% Tween-80.

Sample termites: *Coptotermes formosanus Shiraki*

### ① Toxicity test in the laboratory

15 glass beakers are used in the test. A piece of filter paper was put at the bottom of the beaker and moistened with cotton balls.100 Sample termites were put into the beaker and adding to 1ml suspension with the concentration of 3×10⁷ - 3 × 10⁸spore/ml, respectively. To the control group, 1ml 0.05% Tween-80 was added. Each test shall be repeated for 5 times. The results were shown in Table 4:

**Table 4: the result of toxicity test of Metarhizium anisopliae var. dcjhyium Lj01 vs. Coptotermes formosanus Shiraki**

| Treatment | Termite mortality ( % ) | | | |
|---|---|---|---|---|
| | 1day | 2 days | 3 days | 4 days |
| 0.05% Tween-80 ( control ) | 1.0 | 2.0 | 3.6 | 5.0 |
| 3×10⁷spore/ml | 42.6 | 75.5 | 100.0 | |
| 3×10⁸spore/ml | 57.7 | 100.0 | | |

The results show that, *Metarhizium anisopliae var. dcjhyium* Lj01with the concentration of 3×10⁸ spore/ml possesses excellent killing effect to *Coptotermes formosanus Shiraki.*

### ②Outdoor Toxicity test

Selecting 3 formicaries of C. *formosanus* in the field and digging a hole on the formicaries. To each formicary, 100 ml 3×10⁸ spore/ml *Metarhizium anisopliae var. dcjhyium* Lj01 spore suspension was injected and then, recover the formicary. After 4-6 weeks, digging up the formicaries, it is found that the whole formicary has been filled up with the hypha of the *Metarhizium anisopliae var. dcjhyium* Lj01. Because of the formation of spore, all the termites in the formicary are killed.

### Example 4:

Sample strains: *Metarhizium anisopliae var. dcjhyium* Lj01 was cultured in the improved PDA medium for 10 days under the constant temperature of 28 . The spore was diluted to 3×10⁷ - 3×10⁸ spore/ml using 0.05% Tween-80.

Sample termites: *Reticulitermes chinensis Snyder*

### Toxicity test in the laboratory

15 glass beakers are used in the test. A piece of filter paper was put at the bottom of each beaker and moistened with cotton balls.100 Sample termites were put into the beaker and adding to 1ml suspension with the concentration of 3×10⁷-3×10⁸spore/ml, respectively. To the control group, 1ml 0.05% Tween-80 was added. Each test shall be repeated for 5 times. The results were shown in Table 5:

**Table 5: the result of toxicity test of Metarhizium anisopliae var. dcjhyium Lj01 vs. Reticulitermes chinensis Snyder**

| Treatment | Termite mortality ( % ) | | | |
|---|---|---|---|---|
| | 1day | 2 days | 3 days | 4 days |
| 0.05% Tween-80 ( control ) | 0.0 | 0.5 | 2.0 | 2.5 |
| 3×10⁷spore/ml | 21.9 | 48.7 | 69.4 | 100.0 |
| 3×10⁸spore/ml | 35.9 | 89.4 | 100.0 | |

The results show that, *Metarhizium anisopliae var. dcjhyium* Lj01 with the concentration of 3×10⁸ spore/ml possesses excellent killing effect to *Reticulitermes chinensis Snyder.*

### ②Outdoor Toxicity test

Selecting 3 formicaries of *Reticulitermes chinensis Snyder* in the field and digging one hole on the formicaries. To each formicary, 100 ml 3×10⁸ spore/ml *Metarhizium anisopliae var. dcjhyium* Lj01 spore suspension was injected and then, recover the formicary. After 4-6 weeks, digging up the formicaries, it is found that the whole formicary has been filled up with the hypha of the *Metarhizium anisopliae var. dcjhyium* Lj01. Because of the formation of spore, all the termites in the formicary are killed.

### Example 5:

Sample strains: *Metarhizium anisopliae var.* dcjhyium Lj01 was cultured in the improved PDA medium for 10 days under the constant temperature of 28 . The spore was diluted to 3×10⁷ - 3×10⁸ spore/ml using 0.05% Tween-80.

Sample termites: *Cryptotermes domesticus* Haviland

### Toxicity test in the laboratory

15 glass beakers are used in the test. A piece of filter paper was put at the bottom of each beaker and moistened with cotton balls.100 Sample termites were put into the beaker and added to 1ml suspension with the concentration of 3×10⁷-3×10⁸spore/ml, respectively. To the control group, 1ml 0.05% Tween-80 was added. Each test shall be repeated for 5 times. The results were shown in Table 6:

**Table 6: the result of toxicity test of Metarhizium anisopliae var. dcjhyium Lj01 vs. Cryptotermes domesticus Haviland**

| Treatment | Termite mortality ( % ) | | | |
|---|---|---|---|---|
| | 1day | 2 days | 3 days | 4 days |
| 0.05% Tween-80 ( control ) | 0.0 | 1.2 | 2.0 | 4.0 |
| 3×10⁷spore/ml | 29.7 | 52.5 | 74.0 | 100.0 |
| 3×10⁸spore/ml | 36.0 | 80.5 | 100.0 | |

The results show that, *Metarhizium anisopliae var. dcjhyium* Lj01 with the concentration of 3×10⁸ spore/ml possesses excellent killing effect to *Cryptotermes domesticus* Haviland.

### ②Outdoor Toxicity test

Selecting 3 formicaries of *Cryptotermes domesticus* Haviland *Snyder* in the field and digging one hole on each of the formicaries. To each formicary, 100 ml 3 ×10⁸ spore/ml *Metarhizium anisopliae var. dcjhyium* Lj01 spore suspension was injected and then, recover the formicary. After 6 weeks, digging up the formicaries, it is found that the whole formicary has been filled up with the hypha of the *Metarhizium anisopliae var. dcjhyium* Lj01. Because of the formation of spore, all the termites in the formicary are killed.

### Example 6:

The controlling effect of *Metarhizium anisopliae var. dcjhyium* Lj01 on *Coptotermes formosanus* Shiraki

Sample strains: *Metarhizium anisopliae var. dcjhyium* Lj01 with the concentration of 3×10⁸ spore/ml.

### Sample termites: Coptotermes formosanus Shiraki

One house that has been used for ten years was attacked by *Coptotermes formosanus* Shiraki. The *Coptotermes formosanus* Shiraki can be found on the wardrobe, wood floor, wood door and the ceiling of the house. The researcher posits 6 - 8 kits on the wardrobe, wood floor, wood door and the ceiling of said house. Those kits comprise 3×10⁸ *Metarhizium anisopliae var.* dcjhyium Lj01 spore, food attractant, stuffing and food adhesive etc. After one month, all of the termites in the house have been killed. It can be seen that the dead body of those termite are covered by the hypha or spore of *Metarhizium anisopliae var. dcjhyium* Lj01.

### Example 7:

Toxicity test of *Metarhizium anisopliae* var. dcjhyium Lj01 vs. *Cryptotermes havilandi* Holmgren

Sample strains: *Metarhizium anisopliae* var. dcjhyium Lj01 was cultured in the improved PDA medium for 10 days under the constant temperature of 28 °C. The spore was diluted to 3×10⁷-3×10⁸spore/ml using 0.05% Tween-80.

Sample termites: *Cryptotermes havilandi Holmgren*

15 glass beakers are used in the test. A piece of filter paper was put at the bottom of each beaker and moistened with cotton balls.50 Sample termites were put into the beaker and adding to 1ml suspension with the concentration of 3×10⁷-3× 10⁸spore/ml, respectively. To the control group, 1ml 0.05% Tween-80 was added. Each test shall be repeated for 5 times. The results were shown in Table 7:

**Table 7: the result of toxicity test of Metarhizium anisopliae var. dcjhyium Lj01 vs. Cryptotermes havilandi Holmgren**

| Treatment | Termite mortality (%) | | | |
|---|---|---|---|---|
| | 1day | 2 days | 3 days | 4 days |
| 0.05% Tween-80 (control) | 0.0 | 0.0 | 2.3 | 3.5 |
| 3×10⁷spore/ml | 27.5 | 46.0 | 68.4 | 100.0 |
| 3×10⁸spore/ml | 35.0 | 78.6 | 100.0 | |

The results show that, *Metarhizium anisopliae var. dcjhyium* Lj01 possesses excellent killing effect to *Cryptotermes havilandi Holmgren*.

### Example 8:

Toxicity test of *Metarhizium anisopliae* var. dcjhyium Lj01 vs. *Nasutitermes moratus* Silvestri

Sample strains: *Metarhizium anisopliae* var. dcjhyium Lj01 was cultured in the improved PDA medium for 10 days under the constant temperature of 28 °C. The spore was diluted to 3×10⁸spore/ml using 0.05% Tween-80.

Sample termites: *Nasutitermes moratus* Silvestri

15 glass beakers are used in the test. A piece of filter paper was put at the bottom of each beaker and moistened with cotton balls. 100 Sample termites were put into the beaker and adding to 1ml suspension with the concentration of 3 × 10⁸spore/ml, respectively. To the control group, 1ml 0.05% Tween-80 was added. Each test shall be repeated for 3 times. The results were shown in Table 8:

**Table 8: the result of toxicity test of Metarhizium anisopliae var. dcjhyium Lj01 vs. Nasutitermes moratus Silvestri**

| Treatment | Termite mortality (%) | | | |
|---|---|---|---|---|
| | 1day | 2 days | 3 days | 4 days |
| 0.05% Tween-80 (control) | 0.0 | 0.0 | 1.3 | 1.8 |
| 3×10⁸spore/ml | 21.7 | 69.6 | 95.0 | 100.0 |

The results show that, *Metarhizium anisopliae var. dcjhyium* Lj01 possesses excellent killing effect to *Nasutitermes moratus* Silvestri.

### Example 9:

Toxicity test of *Metarhizium anisopliae* var. dcjhyium Lj01 vs. *Reticulitermes flavipes* Kollar

Sample strains: *Metarhizium anisopliae* var. dcjhyium Lj01 was cultured in the improved PDA medium for 10 days under the constant temperature of 28 °C. The spore was diluted to 3×10⁷-3×10⁸spore/ml using 0.05% Tween-80.

Sample termites: *Reticulitermes flavipes* Kollar

15 glass beakers are used in the test. A piece of filter paper was put at the bottom of each beaker and moistened with cotton balls.100 Sample termites were put into the beaker and adding to 1ml suspension with the concentration of 3×10⁷-3× 10⁸spore/ml, respectively. To the control group, 1ml 0.05% Tween-80 was added. Each test shall be repeated for 3 times. The results were shown in Table 9:

**Table 9: the result of toxicity test of Metarhizium anisopliae var. dcjhyium Lj01 vs. Reticulitermes flavipes Kollar**

| Treatment | Termite mortality (%) | | | |
|---|---|---|---|---|
| | 1day | 2 days | 3 days | 4 days |
| 0.05% Tween-80 (control) | 0.0 | 0.0 | 1.8 | 2.4 |
| 3×10⁷spore/ml | 19.5 | 43.9 | 72.3 | 100.0 |
| 3×10⁸spore/ml | 36.7 | 87.0 | 100.0 | |

The results show that, *Metarhizium anisopliae var. dcjhyium* Lj01 possesses excellent killing effect to *Reticulitermes flavipes* Kollar.

### Example 10:

Toxicity test of *Metarhizium anisopliae* var. dcjhyium Lj01 vs. *Solenopsis invicta*

In latin, "*Solenopsis invicta*" means " invincible ant" which mainly attack to human or other animals and hard to be prevented. *Solenopsis invicta* always act in a large population. When initiating an attack, *solenopsis invicta* will repeatly attack the same target. Some pimple-like red spots will appear on the attacked areas of the target's body and feel like being burned. In USA, *solenopsis invicta* cause the loss of over 1 billion pounds each year. Since 1930s, 84 persons have been killed by *solenopsis invicta* in the south of USA. Solenopsis invicta also be found in southern China, which seriously threatens the safety of people and livestock.

Sample strains: *Metarhizium anisopliae* var. dcjhyium Lj01 was cultured in the improved PDA medium for 10 days under the constant temperature of 28 °C. The spore was diluted to 3×10⁸spore/ml using 0.05% Tween-80.

Sample termites: *Solenopsis invicta*

4 formicaries of *Solenopsis invicta* are selected from the south of china (such as, from Shenzhen) which indulge in wilful persecution of *Solenopsis invicta* and shovelled into 4 plastic tanks respectively. Each tank contains one formicary comprising about 18-20 thousands of *Solenopsis invicta*. To each tank, 200 ml 3× 10⁸spore/ml *Metarhizium anisopliae* var. dcjhyium Lj01 spore suspension was added. The tanks were covered by compact wire netting to prevent the *Solenopsis invicta* escaping from the tank. A smaller plastic tank was adopted to conduct the control test, in which only *Solenopsis invicta* and the earth from the formicary were added without any *Metarhizium anisopliae* var. *dcjhyium* Lj01 spore suspension. 200ml sterilized 0.05% Tween - 80 was added as the control. The tank was also covered by compact wire netting. Both the test and control group are cultured under room temperature (25 °C).

The results show that, *Metarhizium anisopliae var. dcjhyium* Lj01 possesses excellent killing effect to *Reticulitermes flavipes* Kollar.

Result: It was found that, from the sixth day, some *Solenopsis invicta* in test groups were dead and the hypha or spore of *Metarhizium anisopliae* var. dcjhyium Lj01 begin to appear on the earth; on the ninth day, a large number of *Solenopsis invicta* in test groups were dead and the hypha or spore of *Metarhizium anisopliae* var. dcjhyium grow thriftily in the test tanks; and on the thirteen day, all of *Solenopsis invicta* in test groups were dead. However, all of *Solenopsis invicta* in control groups were still alive. Therefore, *Metarhizium anisopliae* var. *dcjhyium* Lj01 possess excellent effect on killing *Solenopsis invicta*.

### Example 11

Toxicity test of *Metarhizium anisopliae* var. dcjhyium Lj01 vs. locust

Sample strains: *Metarhizium anisopliae* var. dcjhyium Lj01was cultured in the improved PDA medium for 10 days under the constant temperature of 28 °C. The spore was diluted to 3×10⁷-3×10⁸spore/ml using 0.05% Tween-80.

Sample pest: locust

9 plastic tanks are used in the test. A piece of filter paper was put at the bottom of each tank and moistened with cotton balls.30 locust were put into the tank and adding to 10ml spore suspension with the concentration of 3×10⁷- 3×10⁸spore/ml, respectively. To the control group, 10ml 0.05% Tween-80 was added. Each test shall be repeated for 3 times. The results were shown in Table 10:

**Table 10: the result of toxicity test of Metarhizium anisopliae var. dcjhyium Lj01vs. locust**

| Treatment | mortality (%) | | | | |
|---|---|---|---|---|---|
| | 3day | 6 days | 9days | 12days | 15days |
| 0.05% Tween-80 (control) | 0.0 | 0.0 | 1.8 | 2.3 | 3.0 |
| 3×10⁷spore/ml | 0.0 | 15.6 | 39.7 | 88.0 | 100.0 |
| 3×10⁸spore/ml | 3.4 | 32.5 | 77.4 | 100.0 | |

The result shows that, *Metarhizium anisopliae* var. *dcjhyium* Lj01 possesses excellent effect on killing locust. Treated with 10 ml 3×10⁸ spore /ml *Metarhizium anisopliae* var. *dcjhyium* Lj01, the mortality of locust on the ninth day is 77.4%, on twelfth day is 100%.

### Example 12

Toxicity test of *Metarhizium anisopliae* var. dcjhyium Lj01 vs. *Dendrolimus*

Sample strains: *Metarhizium anisopliae* var. dcjhyium Lj01 was cultured in the improved PDA medium for 10 days under the constant temperature of 28 °C. The spore was diluted to 3×10⁸ spore/ml using 0.05% Tween-80.

Sample pest: *Dendrolimus*

6 plastic tanks are used in the test. Some fresh pine branches were put at the bottom of each tank.30 Dendrolimus were put into the tank and adding to 10ml spore suspension with the concentration of 3×10⁸spore/ml, respectively. To the control group, 10ml 0.05% Tween-80 was sprayed on the pine needle. Each test shall be repeated for 3 times. The results were shown in Table 11:

**Table 11: the result of toxicity test of Metarhizium anisopliae var. dcjhyium Lj01vs. Dendrolimus**

| Treatment | Mortality (%) | | | |
|---|---|---|---|---|
| | 3day | 6days | 9 days | 12 days |
| 0.05% Tween-80 (control) | 0.0 | 0.0 | 2.1 | 3.2 |
| 3×10⁸spore/ml | 8.9 | 41.3 | 94.6 | 100.0 |

The result shows that *Metarhizium anisopliae* var. dcjhyium Lj01 possess excellent effect on killing Dendrolimus. After treated by 10ml 3×10⁸ spore/ml spore suspension, the mortality of Dendrolimus reaches 94.6%.

### Example 13:

Toxicity test of *Metarhizium anisopliae* var. dcjhyium Lj01 vs. *Blattella germanica*

Sample strains: *Metarhizium anisopliae* var. dcjhyium Lj01 was cultured in the improved PDA medium for 10 days under the constant temperature of 28 °C. The spore was diluted to 3×10⁸ spore/ml using 0.05% Tween-80.

Sample pest: *Blattella germanica*

6 kegs (with the cover for aeration) were selected to conduct the test. At the bottom of each keg, a piece of filter paper was put and moistened with cotton balls. To each keg, 30 cockroaches (*Blattella germanica*) and 2 pieces of fresh flayed potato were added. To the test groups, 10 ml 3×10⁸ spore/ml *Metarhizium anisopliae* var. dcjhyium Lj01 spore suspension were dropped, and to the control groups, 10ml 0.05% Tween-80 were dropped. Each test shall be repeated for 3 times. Observed at regular intervals, the mortality of cockroach was shown in Table 12:

**Table 12: the result of toxicity test of Metarhizium anisopliae var. dcjhyium Lj01 vs. cockroach**

| Treatment | mortality (%) | | | | |
|---|---|---|---|---|---|
| | 9day | 11 days | 13 drays | 15days | 17days |
| 0.05% Tween-80 (control) | 0.0 | 0.0 | 0.0 | 0.0 | 0.0 |
| 3×10⁸spore/ml | 0.0 | 6.7 | 24.5 | 75.0 | 100.0 |

Seen from the result, *M. anisopliae var.dcjhyium* Lj01 possesses well effect on killing blattella germanica and thereby can be used in biological control of cockroach.

While the invention has been described in conjunction with the detailed description thereof, the foregoing description is intended to illustrate and not limit the scope of the invention, which is defined by the scope of the appended claims. Other aspects, advantages, and modifications are within the scope of the following claims. It will be understood by those skilled in the art that various changes in form and details may be made therein without departing from the scope of the invention encompassed by the appended claims. Meanwhile, the non-described inventions, which can be anticipated from this application, are also within the scopes of this application. The applicant remains the right to claim those in the future.

## Claims

1. An isolated substantially homogeneous *Meterhizium anisopliae* ***var*.** *dcjhyium* Lj01, comprising the characteristics as deposition number of CCTCC M206077.

2. The *Meterhizium anisopliae* ***var*.** *dcjhyium* Lj01 of claim 1, wherein the *Meterhizium anisopliae* ***var*.** *dcjhyium* Lj01 is an entomopathogenic fungus.

3. The *Meterhizium anisopliae* ***var*.** *dcjhyium* Lj01 of claim 1, wherein the *Meterhizium anisopliae* ***var*.** *dcjhyium* Lj01 is an entomopathogenic for *Reticulitermes chinensis* **Snyder.**

4. The *Meterhizium anisopliae* ***var*.** *dcjhyium* Lj01 of claim 1, wherein the *Meterhizium anisopliae* ***var*.** *dcjhyium* Lj01 is an entomopathogenic for *Coptotermes formosanus* **Shiraki..**

5. The *Meterhizium anisopliae* ***var*.** *dcjhyium* Lj01 of claim 1, wherein the *Meterhizium anisopliae* ***var*.** *dcjhyium* Lj01 is a entomopathogenic for *Odontotermes formesanus* **Shiraki.**

6. The *Meterhizium anisopliae **var.** dcjhyium* Lj01 of claim 1, wherein the *Meterhizium anisopliae **var.** dcjhyium* Lj01 is an entomopathogenic for one of the inserts selected from the group consisting of *Macrotermes barneyi* Light, *Cryptotermes domesticus* Haviland, *Kalotermes flavicollis* Fabr., *Cryptotermes brevis* Walker, *Reticulitermes santonensis* Feytaud, *Heterotermes indicola* Wasmann, *Reticulitermes flavipes* Kollar, *Reticulitermes hesperus* Banks, *Zootermopsis angusticollis* Hagen, *Nasutitermes moratus* Silvestri, *Solenopsis invicta,* Carpenter ants, Pharoach ants, Locust, *Dendrolimus,* cockroach.

7. An insect-killing effective amount of *M. anisopliae **var.** dcjhyium* Lj01, wherein *M. anisopliae **var.** dcjhyium* Lj01 is isolated from their natural habitat, and cultured, the deposit number of the *M. anisopliae **var.** dcjhyium* Lj01 is CCTCC M206077.

8. The insect-killing effective amount of *M. anisopliae **var.** dcjhyium* Lj01 of claim 7, wherein it is an entomopathogenic fungus.

9. The insect-killing effective amount of *M. anisopliae **var.** dcjhyium* Lj01 of claim 7, wherein the *Meterhizium anisopliae **var.** dcjhyium* Lj01 is an entomopathogenic for *Reticulitermes chinensis* **Snyder.**

10. The insect-killing effective amount of *M. anisopliae **var.** dcjhyium* Lj01 of claim 7, wherein the *Meterhizium anisopliae **var.** dcjhyium* Lj01 is an entomopathogenic for *Coptotermes formosanus* **Shiraki..**

11. The insect-killing effective amount of *M. anisopliae **var.** dcjhyium* Lj01 of claim 7, wherein the *Meterhizium anisopliae **var.** dcjhyium* Lj01 is an entomopathogenic for *Odontotermes formesanus* **Shiraki.**

12. The insect-killing effective amount of *M. anisopliae **var.** dcjhyium* Lj01 of claim 7, wherein the *Meterhizium anisopliae **var.** dcjhyium* Lj01 is a entomopathogenic for one of the insert selected from the group consisting of *Macrotermes barneyi* Light, *Cryptotermes domesticus* Haviland, *Kalotermes flavicollis* Fabr., *Cryptotermes brevis* Walker, *Reticulitermes santonensis* Feytaud, *Heterotermes indicola* Wasmann, *Reticulitermes flavipes* Kollar, *Reticulitermes hesperus* Banks, *Zootermopsis angusticollis* Hagen, *Nasutitermes moratus* Silvestri, *Solenopsis invicta*, Carpenter ants, Pharoach ants, Locust, *Dendrolimus,* cockroach.

13. A bio-pesticides comprising insect-killing effective amount of *M. anisopliae **var.** dcjhyium* Lj01 of any one of claims 1-12.

14. The bio-pesticides of claim 13, wherein said *M. anisopliae **var.** dcjhyium* Lj01 can be the fungi, spores, mycelia, or any combination thereof.

15. The bio-pesticides of claim 13 or claim 14, further comprises an insect pheromones.

16. The bio-pesticides of claim 15, further comprises a food attractant of insects.

17. The bio-pesticides of claim 16, further comprises an adjuvant.

18. A method for killing insect, wherein said insect is one of : *Coptotermes formosanus* Shiraki, *Reticulitermes chinensis* Snyder, *Odontotermes formesanus* Shiraki, *Macrotermes barneyi* Light, *Cryptotermes domesticus* Haviland, *Kalotermes flavicollis* Fabr., *Cryptotermes brevis* Walker, *Reticulitermes santonensis* Feytaud, *Heterotermes indicola* Wasmann, *Reticulitermes flavipes* Kollar, *Reticulitermes hesperus* Banks, *Zootermopsis angusticollis* Hagen, *Nasutitermes moratus* Silvestri, *Solenopsis invicta*, Carpenter ants, Pharoach ants, Locust, *Dendrolimus*, and cockroach, wherein the method comprising use the bio-pesticides comprising insect-killing effective amount of *M. anisopliae **var.** dcjhyium* Lj01 of claim 13 to the habitats of the insects.

19. The method for killing insect of claim 18, wherein said *M. anisopliae **var.** dcjhyium* Lj01 can be the fungi, spores, mycelia, or any combination thereof.

20. A use of *M. anisopliae **var.** dcjhyium* Lj01 (deposit number: CCTCC M206077) in manufacturing an insect-killing agent.

21. An insect-killing device, substantially with a long cylinder shape, wherein said long cylinder shape comprises:
acute terminal, wherein said acute terminal substantially is cuniform;
non-acute- terminal, wherein said non-acute- terminal has a hollow part.

22. The insect-killing device of claim 21, wherein said hollow part comprises the bio-pesticides of any of claims 13-17.
